# EUROPEAN PATENT SPECIFICATION

(11) **EP 4 263 475 B1**
(45) Date of publication and mention of the grant of the patent: **08.01.2025**
(21) Application number: 21840349.1
(22) Date of filing: 14.12.2021
(51) Int. Cl.: C07C 2/32, C07C 2/34, C07C 11/02

(54) **A PROCESS FOR PRODUCING ALPHA-OLEFINS**
VERFAHREN ZUR HERSTELLUNG VON ALPHA-OLEFINEN
PROCÉDÉ DE PRODUCTION D'ALPHA-OLÉFINES

(30) Priority: 15.12.2020 US 202063125713 P
(43) Date of publication of application: 25.10.2023
(73) Proprietor: Shell Internationale Research Maatschappij B.V., 2596 HR The Hague (NL)
(72) Inventor: KOMPLIN, Glenn Charles, Houston, Texas 77082-3101 (US); HUH, Heejae, Houston, Texas 77082-3101 (US); WARD, Gregory John, Houston, Texas 77082-3101 (US)
(74) Representative: Shell Legal Services IP
(86) International application number: PCT/US2021/063261
(87) International publication number: WO 2022/132734

(56) References cited:
- WO-A1-2013/168103
- WO-A1-2019/182664
- US-A1- 2005 131 262
- US-A1- 2014 316 087

## Description

### Field of the Invention

The invention relates to a process for producing alpha-olefins comprising oligomerizing ethylene in the presence of oxygen.

### Background

The oligomerization of olefins, such as ethylene, produces butene, hexene, octene, and other valuable linear alpha olefins. Linear alpha olefins are a valuable comonomer for linear low-density polyethylene and high-density polyethylene. Such olefins are also valuable as a chemical intermediate in the production of plasticizer alcohols, fatty acids, detergent alcohols, polyalphaolefins, oil field drilling fluids, lubricant oil additives, linear alkylbenzenes, alkenylsuccinic anhydrides, alkyldimethylamines, dialkylmethylamines, alpha-olefin sulfonates, internal olefin sulfonates, chlorinated olefins, linear mercaptans, aluminum alkyls, alkyldiphenylether disulfonates, and other chemicals.

US 6,683,187 describes a bis(arylimino)pyridine ligand, catalyst precursors and catalyst systems derived from this ligand for ethylene oligomerization to form linear alpha olefins. The patent teaches the production of linear alpha olefins with a Schulz-Flory oligomerization product distribution. In such a process, a wide range of oligomers are produced, and the fraction of each olefin can be determined by calculation on the basis of the K-factor. The K-factor is the molar ratio of (Cₙ+2)/Cₙ, where n is the number of carbons in the linear alpha olefin product.

US 7,304,159 describes a process for the oligomerization of ethylene to linear alpha olefins. The patent teaches the treatment of the ethylene to reduce water and oxygen to less than 1 ppm. Further, CN 102850168 describes an ethylene oligomerization process, and it teaches the removal of water, oxygen and catalyst poisons. US 10,160,696 also teaches that the oligomerization is typically carried out under conditions that substantially exclude oxygen, water and other materials that act as catalyst poisons. The patent further teaches that the reactor is purged with nitrogen or argon before introducing catalyst into the reactor.

US2014/316087 describes a catalyst composition for ethylene oligomerization including an imino ferrous complex. The publication teaches a high oligomerization activity can be obtained using said imino ferrous complex (described therein) as the main catalyst, an aluminum-containing cocatalyst, water, and an organic solvent.

WO 2013/168103 describes a process for oligomerisation of an olefinic compound for producing an oligomeric product carried out in the presence of an activated catalyst, a non-metal oxygen containing additive and optionally a zinc compound.

It would be advantageous to develop an improved process that would provide an increased production of alpha-olefins with an oligomerization product distribution having a desired K-factor and product quality.

### Summary of the Invention

The invention provides a process for producing alpha-olefins comprising contacting an ethylene feed with an oligomerization catalyst system in an oligomerization reaction zone under oligomerization reaction conditions to produce a product stream comprising alpha-olefins wherein the catalyst system comprises an iron-ligand complex and a co-catalyst and the oligomerization reaction zone comprises oxygen at a molar ratio of oxygen to iron of from 1:1 to 200:1.

The invention further provides a process for producing alpha-olefins comprising contacting an ethylene feed with an oligomerization catalyst system in an oligomerization reaction zone under oligomerization reaction conditions to produce a product stream comprising alpha-olefins wherein the catalyst system comprises an iron-ligand complex and a co-catalyst comprising aluminum and the oligomerization reaction zone comprises oxygen at a molar ratio of oxygen to aluminum in MMAO of less than 1:5.

### Brief Description of the Drawings

Figure 1 depicts the alpha olefin production rate in a pilot plant experiment described in Example 1.
Figure 2 depicts the alpha olefin production rate in a pilot plant experiment described in Example 2.
Figure 3 depicts the alpha olefin production rate in a pilot plant experiment described in Example 3.
Figure 4 depicts the pilot plant configuration used in the Examples.

### Detailed Description

The process comprises converting an olefin feed into a higher oligomer product stream by contacting the feed with an oligomerization catalyst system and a co-catalyst in an oligomerization reaction zone under oligomerization conditions. In one embodiment, an ethylene feed may be contacted with an iron-ligand complex and modified methyl aluminoxane under oligomerization conditions to produce a product slate of alpha olefins having a specific k-factor.

The process comprises conducting the oligomerization reaction in the presence of oxygen. It has been found that the addition of oxygen, in a preferred embodiment by adding the oxygen to the ethylene stream, contrary to prior art teachings, actually improves the production of alpha-olefins in the oligomerization process as described herein.

### Olefin Feed

The olefin feed to the process comprises ethylene. The feed may also comprise olefins having from 3 to 8 carbon atoms. The ethylene may be pretreated to remove impurities, especially impurities that impact the reaction, product quality or damage the catalyst. In one embodiment, the ethylene may be dried to remove water. Any pretreatment method known to one of ordinary skill in the art can be used to pretreat the feed.

### Oxygen

The prior art teaches the removal of oxygen from the ethylene feed and from the reactor before the introduction of catalyst. Contrary to that teaching, it has been found that the process operates better in the presence of oxygen. The oxygen may be added to the reactor in any manner known to one of skill in the art. The oxygen may be fed in the presence of other gases, for example nitrogen. In one embodiment, air is fed to the reactor.

The oligomerization reaction zone comprises oxygen at a molar ratio of oxygen to iron of from 1:1 to 200:1. The reaction zone preferably comprises oxygen at a molar ratio of oxygen to iron of from 1.5:1 to 100:1, more preferably from 2:1 to 50:1, even more preferably from 2:1 to 20:1, and most preferably from 2:1 to 6:1. The reaction zone may comprise oxygen at a molar ratio of oxygen to iron of from 3:1 to 6:1.

In one embodiment, the ethylene feed comprises an amount of oxygen sufficient to provide the concentration of oxygen in the reaction zone. In another embodiment, oxygen is added to the ethylene feed to provide oxygen to the reaction zone. In another embodiment, oxygen is fed separately to the reaction zone.

In one embodiment, the oxygen is combined with the iron-ligand complex before it is fed to the reaction zone. In another embodiment, the oxygen is combined with the co-catalyst before it is fed to the reaction zone.

The oligomerization reaction zone may comprise oxygen at a molar ratio of oxygen to aluminum in MMAO of less than 1:5. The reaction zone preferably comprises oxygen at a molar ratio of oxygen to aluminum in MMAO of from 1:5 to 1:20.

### Oligomerization Catalyst

The oligomerization catalyst system may comprise one or more oligomerization catalysts as described further herein. The oligomerization catalyst is a metal-ligand complex that is effective for catalyzing an oligomerization process. The ligand may comprise a bis(arylimino)pyridine compound, a bis(alkylimino)pyridine compound or a mixed aryl-alkyl iminopyridine compound.

### Ligand

In one embodiment, the ligand comprises a pyridine bis(imine) group. The ligand may be a bis(arylimino)pyridine compound having the structure of Formula I.

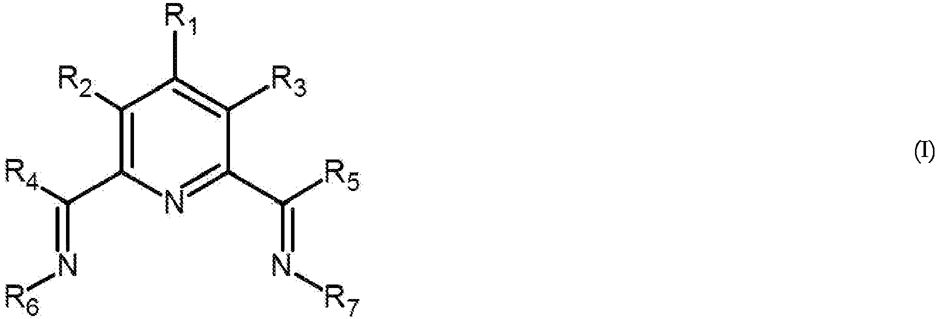

R₁, R₂ and R₃ are each independently hydrogen, optionally substituted hydrocarbyl, hydroxo, cyano or an inert functional group. R₄ and R₅ are each independently hydrogen, optionally substituted hydrocarbyl, hydroxo, cyano or an inert functional group. R₆ and R₇ are each independently an aryl group as shown in Formula II. The two aryl groups (R₆ and R₇) on one ligand may be the same or different.

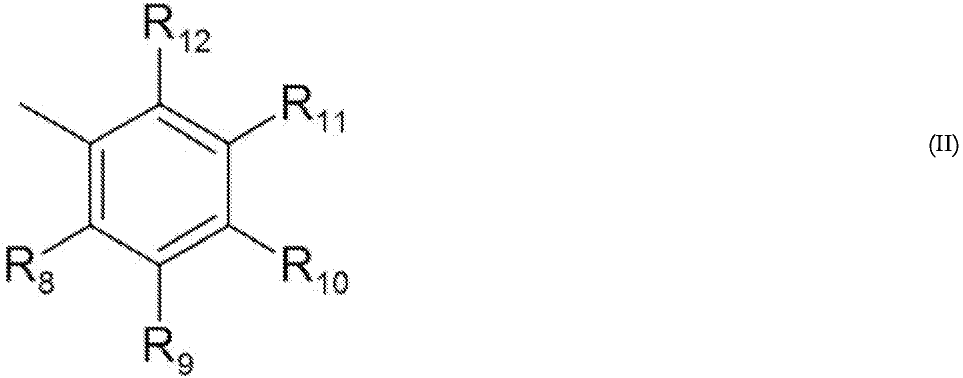

R₈, R₉, R₁₀, R₁₁, R₁₂ are each independently hydrogen, optionally substituted hydrocarbyl, hydroxo, cyano, an inert functional group, fluorine, or chlorine. Any two of R₁-R₃, and R₉-R₁₁ vicinal to one another taken together may form a ring. R₁₂ may be taken together with R₁₁, R₄ or R₅ to form a ring. R₂ and R₄ or R₃ and R₅ may be taken together to form a ring.

A hydrocarbyl group is a group containing only carbon and hydrogen. The number of carbon atoms in this group is preferably in the range of from 1 to 30.

An optionally substituted hydrocarbyl is a hydrocarbyl group that optionally contains one or more "inert" heteroatom-containing functional groups. Inert means that the functional groups do not interfere to any substantial degree with the oligomerization process. Examples of these inert groups include fluoride, chloride, iodide, stannanes, ethers, hydroxides, alkoxides and amines with adequate steric shielding. The optionally substituted hydrocarbyl group may include primary, secondary and tertiary carbon atoms groups.

Primary carbon atom groups are a -CH₂-R group wherein R may be hydrogen, an optionally substituted hydrocarbyl or an inert functional group. Examples of primary carbon atom groups include -CH₃, -C₂H₅, -CH₂Cl, -CH₂OCH₃, -CH₂N(C₂H₅)2, and -CH₂Ph. Secondary carbon atom groups are a -CH-R₂ or -CH(R)(R')group wherein R and R' may be optionally substituted hydrocarbyl or an inert functional group. Examples of secondary carbon atom groups include - CH(CH₃)₂, -CHCl₂, -CHPh₂, -CH(CH₃)(OCH₃),-CH=CH₂, and cyclohexyl. Tertiary carbon atom groups are a -C-(R)(R')(R") group wherein R, R', and R" may be optionally substituted hydrocarbyl or an inert functional group. Examples of tertiary carbon atom groups include -C(CH₃)₃, -CCl₃, - C≡CPh, 1-Adamantyl, and -C(CH₃)₂(OCH₃)

An inert functional group is a group other than optionally substituted hydrocarbyl that is inert under the oligomerization conditions. Inert has the same meaning as provided above. Examples of inert functional groups include halide, ethers, and amines, in particular tertiary amines.

Substituent variations of R₁-R₅, R₈-R₁₂ and R₁₃-R₁₇ may be selected to enhance other properties of the ligand, for example, solubility in non-polar solvents. A number of embodiments of possible oligomerization catalysts are further described below having the structure shown in Formula 3.

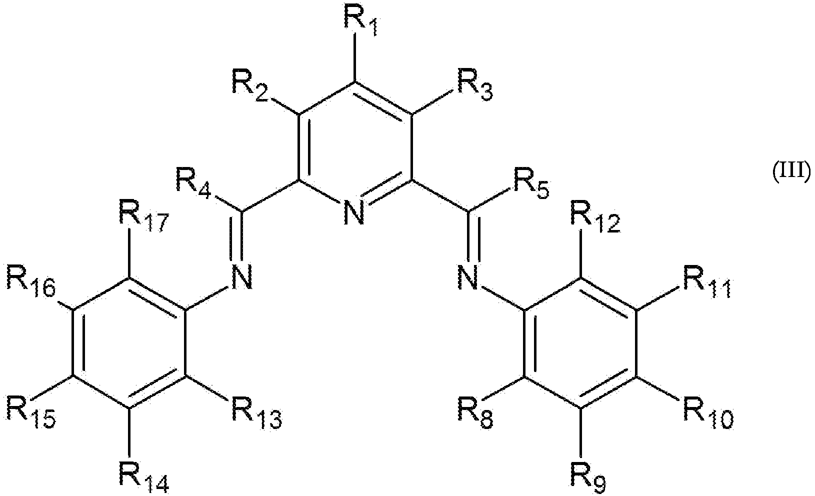

In one embodiment, a ligand of Formula III is provided wherein R₁-R₅, R₉-R₁₁ and R₁₄-R₁₆ are hydrogen; and R₈, R₁₂, R₁₃ and R₁₇ are fluorine.

In one embodiment, a ligand of Formula III is provided wherein R₁-R₅, R₈, R₁₀, R₁₂, R₁₄ and R₁₆ are hydrogen; R₁₃, R₁₅ and R₁₇ are methyl and R₉ and R₁₁ are tert-butyl.

In one embodiment, a ligand of Formula III is provided wherein R₁-R₅, R₈, R₁₂, R₁₄ and R₁₆ are hydrogen; R₁₃, R₁₅ and R₁₇ are methyl; R₉ and R₁₁ are phenyl and R₁₀ is an alkoxy.

In one embodiment, a ligand of Formula III is provided wherein R₁-R₅, Rs, R₁₀, R₁₁ and R₁₄-R₁₆ are hydrogen; R₉ and R₁₂ are methyl; and R₁₃ and R₁₇ are fluorine.

In one embodiment, a ligand of Formula III is provided wherein R₁-R₃, R₉-R₁₁ and R₁₄-Rₗ₆ are hydrogen; R₄ and R₅ are phenyl and R₈, R₁₂, R₁₃ and R₁₇ are fluorine.

In one embodiment, a ligand of Formula III is provided wherein R₁-R₅, R₈-R₉, R₁₁-R₁₂, R₁₃-R₁₄ and R₁₆-R₁₇ are hydrogen; and Rio and R₁₅ are fluorine.

In one embodiment, a ligand of Formula III is provided wherein R₁-R₅, R₈, R₁₀, R₁₂, R₁₃, R₁₅ and R₁₇ are hydrogen; and R₉, R₁₁, R₁₄ and R₁₆ are fluorine.

In one embodiment, a ligand of Formula III is provided wherein R₁-R₅, R₉, R₁₁-R₁₂, R₁₄ and R₁₆-R₁₇ are hydrogen; and R₈, R₁₀, R₁₃ and R₁₅ are fluorine.

In one embodiment, a ligand of Formula III is provided wherein R₁-R₅, R₈-R₉, R₁₁-R₁₂, R₁₄ and R₁₆ are hydrogen; Rio is tert-butyl; and R₁₃, R₁₅ and R₁₇ are methyl.

In one embodiment, a ligand of Formula III is provided wherein R₁-R₅, R₉-R₁₂, R₁₄ and R₁₆ are hydrogen; R₈ is fluorine; and R₁₃, R₁₅ and R₁₇ are methyl.

In one embodiment, a ligand of Formula III is provided wherein R₁-R₅, R₉-R₁₂, R₁₃, R₁₅ and R₁₇ are hydrogen; R₈ is tert-butyl; and R₁₄ and R₁₆ are methyl.

In one embodiment, a ligand of Formula III is provided wherein R₁-R₅, R₉-R₁₂, R₁₃-R₁₄ and R₁₆-R₁₇ are hydrogen; and R₈ and R₁₅ are tert-butyl.

In one embodiment, a ligand of Formula III is provided wherein R₁-R₅, R₈-R₁₀, R₁₃-R₁₄ and R₁₆-R₁₇ are hydrogen; R₁₅ is tert-butyl; and R₁₁, and R₁₂ are taken together to form an aryl group.

In one embodiment, a ligand of Formula III is provided wherein R₁-R₅, R₉-R₁₂, R₁₄-R₁₇ are hydrogen; and R₈ and R₁₃ are methyl.

In one embodiment, a ligand of Formula III is provided wherein R₁-R₅, R₈-R₉, R₁₁-R₁₂, R₁₄ and R₁₆ are hydrogen; Rio is fluorine; and R₁₃, R₁₅ and R₁₇ are methyl.

In one embodiment, a ligand of Formula III is provided wherein R₁-R₅, R₈, R₁₀, R₁₂, R₁₄ and R₁₆ are hydrogen; R₉ and R₁₁ are fluorine; and R₁₃, R₁₅ and R₁₇ are methyl.

In one embodiment, a ligand of Formula III is provided wherein R₁-R₅, R₈-R₉, R₁₁,-R₁₂, R₁₄ and R₁₆ are hydrogen; R₁₀ is an alkoxy; and R₁₃, R₁₅ and R₁₇ are methyl.

In one embodiment, a ligand of Formula III is provided wherein R₁-R₅, R₈-R₉, R₁₁-R₁₂, R₁₄ and R₁₆ are hydrogen; R₁₀ is a silyl ether; and R₁₃, R₁₅ and R₁₇ are methyl.

In one embodiment, a ligand of Formula III is provided wherein R₁-R₅, R₈, R₁₀, R₁₂, R₁₄-R₁₆ are hydrogen; R₉ and R₁₁ are methyl; and R₁₃ and R₁₇ are ethyl.

In one embodiment, a ligand of Formula III is provided wherein R₁-R₅, R₉-R₁₂, and R₁₄-R₁₇ are hydrogen; and R₈ and R₁₃ are ethyl.

In one embodiment, a ligand of Formula III is provided wherein R₁-R₅, R₉-R₁₁ and R₁₄-R₁₆ are hydrogen; and R₈, R₁₂, R₁₃ and R₁₇ are chlorine.

In one embodiment, a ligand of Formula III is provided wherein R₁-R₅, R₉, R₁₁, R₁₄ and R₁₆ are hydrogen; and R₈, R₁₀, R₁₂, R₁₃, R₁₅ and R₁₇ are methyl.

In one embodiment, a ligand of Formula III is provided wherein R₁-R₅, R₉-R₁₀, R₁₂, R₁₄-R₁₅ and R₁₇ are hydrogen; and R₈, R₁₁, R₁₃ and R₁₆ are methyl.

In one embodiment, a ligand of Formula III is provided wherein R₁-R₁₇ are hydrogen.

In one embodiment, a ligand of Formula III is provided wherein R₁-R₅, R₈, R₁₀, R₁₂, R₁₃, R₁₅ and R₁₇ are hydrogen; and R₉, R₁₁, R₁₄ and R₁₆ are tert-butyl.

In one embodiment, a ligand of Formula III is provided wherein R₁-R₅, R₈-R₁₂, R₁₄ and R₁₆ are hydrogen; and R₁₃, R₁₅ and R₁₇ are methyl.

In one embodiment, a ligand of Formula III is provided wherein R₁-R₅, R₉, R₁₁-R₁₂, R₁₄ and R₁₆ are hydrogen; R₈ and R₁₀ are fluorine; and R₁₃, R₁₅ and R₁₇ are methyl.

In one embodiment, a ligand of Formula III is provided wherein R₁-R₅, R₉, R₁₁-R₁₂, R₁₄ and R₁₆-R₁₇ are hydrogen; and R₈, R₁₀, R₁₃ and R₁₅ are methyl.

In one embodiment, a ligand of Formula III is provided wherein R₁-R₅, R₉-R₁₁ and R₁₄-R₁₆ are hydrogen; R₈ and R₁₂ are chlorine; and R₁₃ and R₁₇ are fluorine.

In one embodiment, a ligand of Formula III is provided wherein R₁-R₅, R₈, R₁₀, R₁₂, R₁₄ and R₁₆ are hydrogen; and R₉, R₁₁, R₁₃, R₁₅ and R₁₇ are methyl.

In one embodiment, a ligand of Formula III is provided wherein R₁-R₅, R₉-R₁₁ and R₁₃-R₁₄ and R₁₆-R₁₇ are hydrogen; R₈ and R₁₂ are chlorine; and R₁₅ is tert-butyl.

In one embodiment, a ligand of Formula III is provided wherein R₁-R₅, R₉-R₁₁ and R₁₃-R₁₇ are hydrogen; and R₈ and R₁₂ are chlorine.

In one embodiment, a ligand of Formula III is provided wherein R₁-R₅, R₉-R₁₂, and R₁₄-R₁₇ are hydrogen; and R₈ and R₁₃ are chlorine.

In one embodiment, a ligand of Formula III is provided wherein R₁-R₅, R₉, R₁₁-R₁₂, R₁₄ and R₁₆-R₁₇ are hydrogen; and R₈, R₁₀, R₁₃ and R₁₅ are chlorine.

In one embodiment, a ligand of Formula III is provided wherein R₁-R₅, R₉, R₁₁-R₁₂, and R₁₄, and R₁₆-R₁₇ are hydrogen; R₁₀ and R₁₅ are methyl; and R₈ and R₁₃ are chlorine.

In one embodiment, a ligand of Formula III is provided wherein R₁-R₅, R₉-R₁₁ and R₁₃-R₁₄ and R₁₆-R₁₇ are hydrogen; R₁₅ is fluorine; and R₈ and R₁₂ are chlorine.

In one embodiment, a ligand of Formula III is provided wherein R₁-R₅, R₈-R₉, R₁₁-R₁₂, R₁₄-R₁₅ and R₁₇ are hydrogen; R₁₀ is tert-butyl; and R₁₃ and R₁₆ are methyl.

In one embodiment, a ligand of Formula III is provided wherein R₁-R₅, R₉-R₁₁, R₁₄ and R₁₆ are hydrogen; R₈ and R₁₂ are fluorine; and R₁₃, R₁₅ and R₁₇ are methyl.

In one embodiment, a ligand of Formula III is provided wherein R₁-R₅, R₉-R₁₀, R₁₂, R₁₄-Rₗ₅ and R₁₇ are hydrogen; R₈ and R₁₃ are methyl; and R₁₁ and R₁₆ are isopropyl.

In one embodiment, a ligand of Formula III is provided wherein R₁-R₅, R₉-R₁₂ and R₁₄-R₁₆ are hydrogen; R₈ is ethyl; and R₁₃ and R₁₇ are fluorine.

In one embodiment, a ligand of Formula III is provided wherein R₂-R₅, R₉-R₁₀, R₁₂, R₁₄-R₁₅ and R₁₇ are hydrogen; R₁ is methoxy; and R₈, R₁₁, R₁₃ and R₁₆ are methyl.

In one embodiment, a ligand of Formula III is provided wherein R₂-R₅, R₈-R₁₂, R₁₄ and R₁₆ are hydrogen; R₁ is methoxy; and R₁₃, R₁₅ and R₁₇ are methyl.

In one embodiment, a ligand of Formula III is provided wherein R₂-R₅, R₉-R₁₂, and R₁₄-R₁₇ are hydrogen; R₁ is methoxy; and R₈ and R₁₃ are ethyl.

In one embodiment, a ligand of Formula III is provided wherein R₂-R₅, R₉, R₁₁-R₁₂, R₁₄ and R₁₆-R₁₇ are hydrogen; R₁ is tert-butyl; and R₈, R₁₀, R₁₃ and R₁₅ are methyl.

In one embodiment, a ligand of Formula III is provided wherein R₂-R₅, R₈-R₁₂, R₁₄ and R₁₆ are hydrogen; R₁ is tert-butyl; and R₁₃, R₁₅ and R₁₇ are methyl.

In one embodiment, a ligand of Formula III is provided wherein R₂-R₅, R₉, R₁₁, R₁₄ and R₁₆ are hydrogen; R₁ is methoxy; and R₈, Rio, R₁₂, R₁₃, R₁₅ and R₁₇ are methyl.

In one embodiment, a ligand of Formula III is provided wherein R₂-R₅, R₉, R₁₁, R₁₄ and R₁₆ are hydrogen; R₁ is alkoxy; and R₈, Rio, R₁₂, R₁₃, R₁₅ and R₁₇ are methyl.

In one embodiment, a ligand of Formula III is provided wherein R₂-R₅, R₉, R₁₁, R₁₄ and R₁₆ are hydrogen; R₁ is tert-butyl; and R₈, R₁₀, R₁₂, R₁₃, R₁₅ and R₁₇ are methyl.

In another embodiment, the ligand may be a compound having the structure of Formula I,
wherein one of R₆ and R₇ is aryl as shown in Formula II and one of R₆ and R₇ is pyridyl as shown in Formula IV. In another embodiment, R₆ and R₇ may be pyrrolyl.

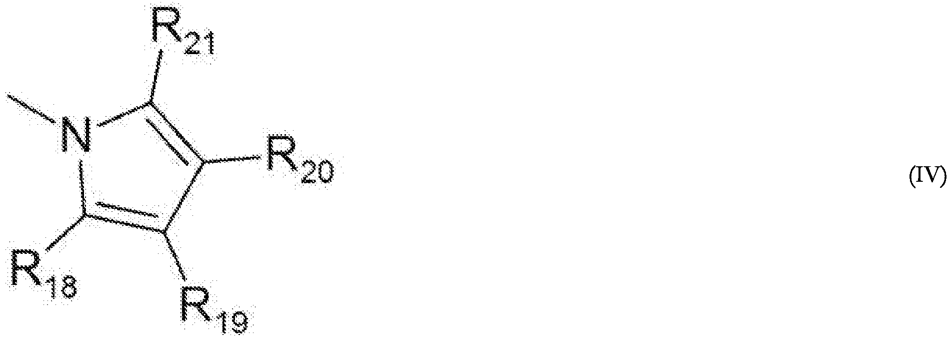

R₁, R₂ and R₃ are each independently hydrogen, optionally substituted hydrocarbyl, hydroxo, cyano or an inert functional group. R₄ and R₅ are each independently hydrogen, optionally substituted hydrocarbyl, hydroxo, cyano or an inert functional group. R₈-R₁₂ and R₁₈-R₂₁ are each independently hydrogen, optionally substituted hydrocarbyl, hydroxo, cyano, an inert functional group, fluorine, or chlorine. Any two of R₁-R₃, and R₉-R₁₁ vicinal to one another taken together may form a ring. R₁₂ may be taken together with R₁₁, R₄ or R₅ to form a ring. R₂ and R₄ or R₃ and R₅ may be taken together to form a ring.

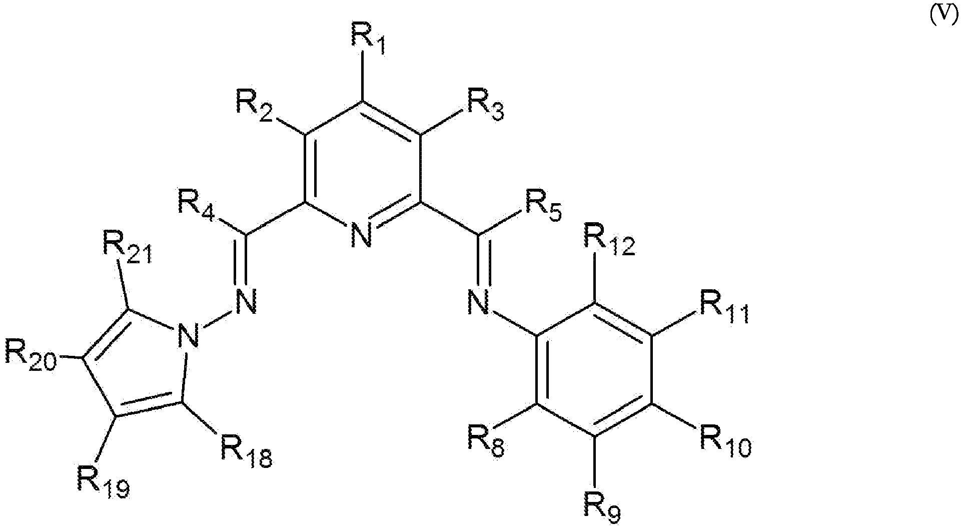

In one embodiment, a ligand of Formula V is provided wherein R₁-R₅, R₉, R₁₁ and R₁₈-R₂₁ are hydrogen; and R₈, R₁₀, and R₁₂ are methyl.

In one embodiment, a ligand of Formula V is provided wherein R₁-R₅, R₉-R₁₁ and R₁₈-R₂₁ are hydrogen; and R₈ and R₁₂ are ethyl.

In another embodiment, the ligand may be a compound having the structure of Formula I, wherein one of R₆ and R₇ is aryl as shown in Formula II and one of R₆ and R₇ is cyclohexyl as shown in Formula VI. In another embodiment, R₆ and R₇ may be cyclohexyl.

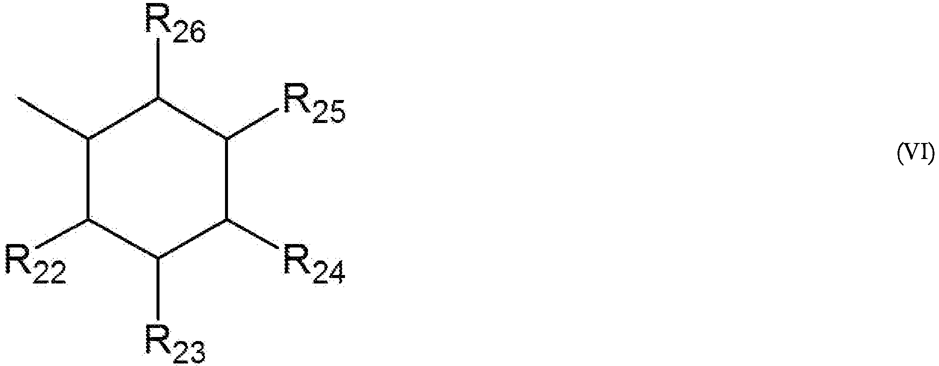

R₁, R₂ and R₃ are each independently hydrogen, optionally substituted hydrocarbyl, hydroxo, cyano or an inert functional group. R₄ and R₅ are each independently hydrogen, optionally substituted hydrocarbyl, hydroxo, cyano or an inert functional group. R₈-R₁₂ and R₂₂-R₂₆ are each independently hydrogen, optionally substituted hydrocarbyl, hydroxo, cyano, an inert functional group, fluorine, or chlorine. Any two of R₁-R₃, and R₉-R₁₁ vicinal to one another taken together may form a ring. R₁₂ may be taken together with R₁₁, R₄ or R₅ to form a ring. R₂ and R₄ or R₃ and R₅ may be taken together to form a ring.

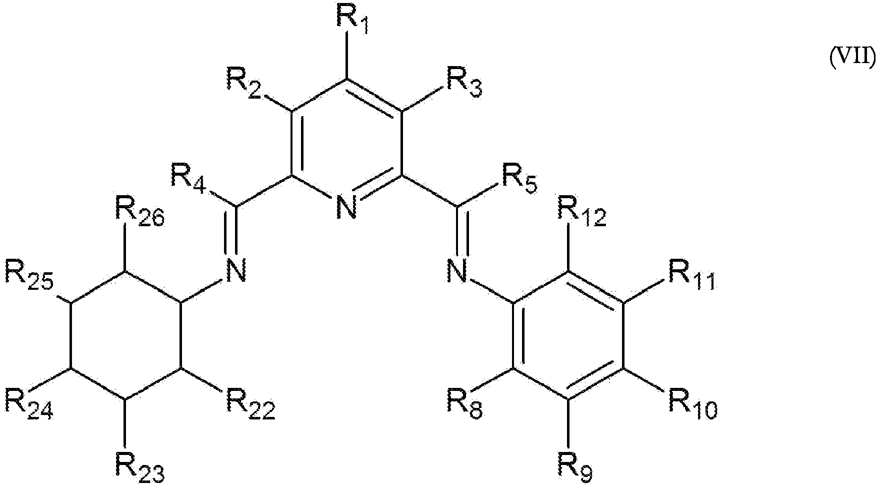

In one embodiment, a ligand of Formula VII is provided wherein R₁-R₅, R₉, R₁₁ and R₂₂-R₂₆ are hydrogen; and R₈, R₁₀, and R₁₂ are methyl.

In another embodiment, R₆ and R₇ may be adamantyl or another cycloalkane.

In another embodiment, the ligand may be a compound having the structure of Formula I, wherein one of R₆ and R₇ is aryl as shown in Formula II and one of R₆ and R₇ is ferrocenyl as shown in Formula VIII. In another embodiment, R₆ and R₇ may be ferrocenyl.

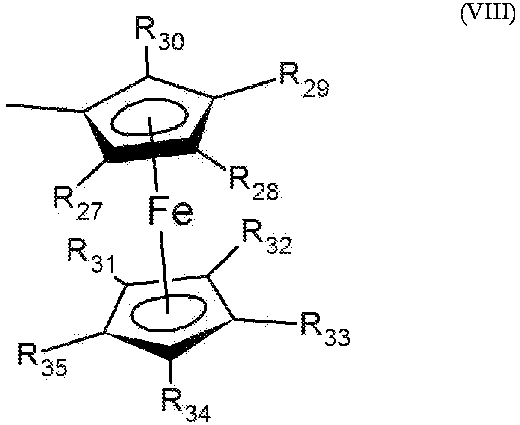

R₁, R₂ and R₃ are each independently hydrogen, optionally substituted hydrocarbyl, hydroxo, cyano or an inert functional group. R₄ and R₅ are each independently hydrogen, optionally substituted hydrocarbyl, hydroxo, cyano or an inert functional group. R₈- R₁₂ and R₂₇ -R₃₅ are each independently hydrogen, optionally substituted hydrocarbyl, hydroxo, cyano, an inert functional group, fluorine, or chlorine. Any two of R₁-R₃, and R₉-R₁₁ vicinal to one another taken together may form a ring. R₁₂ may be taken together with R₁₁, R₄ or R₅ to form a ring. R₂ and R₄ or R₃ and R₅ may be taken together to form a ring. 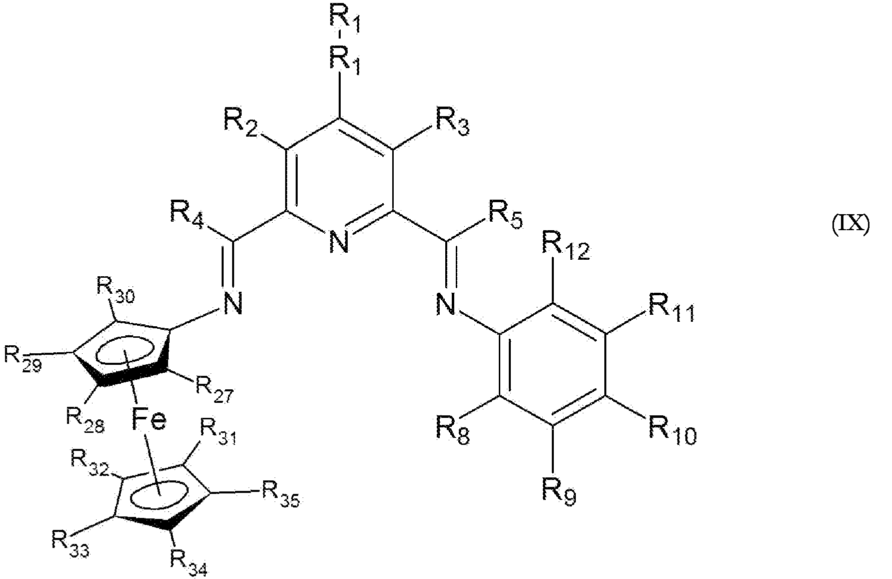 embodiment, a ligand of Formula IX is provided wherein R₁-R₅, R₉, R₁₁ and R₂₇ -R₃₅ are hydrogen; and R₈, R₁₀, and R₁₂ are methyl.

In one embodiment, a ligand of Formula IX is provided wherein R₁-R₅, R₉-R₁₁, and R₂₇ -R₃₅ are hydrogen; and R₈ and R₁₂ are ethyl.

In another embodiment, the ligand may be a bis(alkylamino)pyridine. The alkyl group may have from 1 to 50 carbon atoms. The alkyl group may be a primary, secondary, or tertiary alkyl group. The alkyl group may be selected from the group consisting of methyl, ethyl, propyl, isopropyl, butyl, sec-butyl, isobutyl, and tert-butyl. The alkyl group may be selected from any n-alkyl or structural isomer of an n-alkyl having 5 or more carbon atoms, e.g., n-pentyl; 2-methyl-butyl; and 2,2-dimethylpropyl.

In another embodiment, the ligand may be an alkyl-alkyl iminopyridine, where the two alkyl groups are different. Any of the alkyl groups described above as being suitable for a bis(alkylamino)pyridine are also suitable for this alkyl-alkyl iminopyridine.

In another embodiment, the ligand may be an aryl alkyl iminopyridine. The aryl group may be of a similar nature to any of the aryl groups described with respect to the bis(arylimino)pyridine compound and the alkyl group may be of a similar nature to any of the alkyl groups described with respect to the bis(alkylamino)pyridine compound.

In addition to the ligand structures described hereinabove, any structure that combines features of any two or more of these ligands can be a suitable ligand for this process. Further, the oligomerization catalyst system may comprise a combination of one or more of any of the described oligomerizations catalysts.

The ligand feedstock may contain between 0 and 10 wt% bisimine pyridine impurity, preferably 0-1 wt% bisimine pyridine impurity, most preferably 0-0.1 wt% bisimine pyridine impurity. This impurity is believed to cause the formation of polymers in the reactor, so it is preferable to limit the amount of this impurity that is present in the catalyst system.

In one embodiment, the bisimine pyridine impurity is a ligand of Formula II in which three of R₈, R₁₂, R₁₃, and R₁₇ are each independently optionally substituted hydrocarbyl.

In one embodiment, the bisimine pyridine impurity is a ligand of Formula II in which all four of R₈, R₁₂, R₁₃, and R₁₇ are each independently optionally substituted hydrocarbyl.

### Metal

The metal may be a transition metal, and the metal is preferably present as a compound having the formula MXₙ, where M is the metal, X is a monoanion and n represents the number of monoanions (and the oxidation state of the metal).

The metal can comprise any Group 4-10 transition metal. The metal can be selected from the group consisting of titanium, zirconium, hafnium, vanadium, niobium, tantalum, chromium, molybdenum, tungsten, manganese, iron, cobalt, nickel, palladium, platinum, ruthenium and rhodium. In one embodiment, the metal is cobalt or iron. In a preferred embodiment, the metal is iron. The metal of the metal compound can have any positive formal oxidation state of from 2 to 6 and is preferably 2 or 3.

The monoanion may comprise a halide, a carboxylate, a β-diketonate, a hydrocarboxide, an optionally substituted hydrocarbyl, an amide or a hydride. The hydrocarboxide may be an alkoxide, an aryloxide or an aralkoxide. The halide may be fluorine, chlorine, bromine or iodine.

The carboxylate may be any C₁ to C₂₀ carboxylate. The carboxylate may be acetate, a propionate, a butyrate, a pentanoate, a hexanoate, a heptanoate, an octanoate, a nonanoate, a decanoate, an undecanoate, or a dodecanoate. In addition, the carboxylate may be 2-ethylhexanoate or trifluoroacetate.

The β-diketonate may be any C₁ to C₂₀ β-diketonate. The β-diketonate may be acetylacetonate, hexafluoroacetylacetonate, or benzoylacetonate.

The hydrocarboxide may be any C₁ to C₂₀ hydrocarboxide. The hydrocarboxide may be a C₁ to C₂₀ alkoxide, or a C₆ to C₂₀ aryloxide. The alkoxide may be methoxide, ethoxide, a propoxide (e.g., iso-propoxide) or a butoxide (e.g., tert-butoxide). The aryloxide may be phenoxide

Generally, the number of monoanions equals the formal oxidation state of the metal atom.

Preferred embodiments of metal compounds include iron acetylacetonate, iron chloride, and iron bis(2-ethylhexanoate). In addition to the oligomerization catalyst, a co-catalyst is used in the oligomerization reaction.

### Co-catalyst

The co-catalyst may be a compound that is capable of transferring an optionally substituted hydrocarbyl or hydride group to the metal atom of the catalyst and is also capable of abstracting an X⁻ group from the metal atom M. The co-catalyst may also be capable of serving as an electron transfer reagent or providing sterically hindered counterions for an active catalyst.

The co-catalyst may comprise two compounds, for example one compound that is capable of transferring an optionally substituted hydrocarbyl or hydride group to metal atom M and another compound that is capable of abstracting an X⁻ group from metal atom M. Suitable compounds for transferring an optionally substituted hydrocarbyl or hydride group to metal atom M include organoaluminum compounds, alkyl lithium compounds, Grignards, alkyl tin and alkyl zinc compounds. Suitable compounds for abstracting an X⁻ group from metal atom M include strong neutral Lewis acids such as SbF₅, BF₃ and Ar₃B wherein Ar is a strong electron-withdrawing aryl group such as C₆F₅ or 3,5-(CF₃)₂C₆H₃. A neutral Lewis acid donor molecule is a compound which may suitably act as a Lewis base, such as ethers, amines, sulfides and organic nitrites.

The co-catalyst is preferably an organoaluminum compound which may comprise an alkylaluminum compound, an aluminoxane or a combination thereof.

The alkylaluminum compound may be trialkylaluminum, an alkylaluminum halide, an alkylaluminum alkoxide or a combination thereof. The alkyl group of the alkylaluminum compound may be any C₁ to C₂₀ alkyl group. The alkyl group may be methyl, ethyl, propyl, butyl, pentyl, hexyl, heptyl or octyl. The alkyl group may be an iso-alkyl group.

The trialkylaluminum compound may comprise trimethylaluminum (TMA), triethylaluminum (TEA), tripropylaluminum, tributylaluminum, tripentylaluminum, trihexylaluminum, triheptylaluminum, trioctylaluminum or mixtures thereof. The trialkylaluminum compound may comprise tri-n-propylaluminum (TNPA), tri-n-butylaluminum (TNBA), tri-iso-butylaluminum (TIBA), tri-n-hexylaluminum, tri-n-octylaluminum (TNOA).

The halide group of the alkylaluminum halide may be chloride, bromide or iodide. The alkylaluminum halide may be diethylaluminum chloride, diethylaluminum bromide, ethylaluminum dichloride, ethylaluminum sesquichloride or mixtures thereof.

The alkoxide group of the alkylaluminum alkoxide may be any C₁ to C₂₀ alkoxy group. The alkoxy group may be methoxy, ethoxy, propoxy, butoxy, pentoxy, hexoxy, heptoxy or octoxy. The alkylaluminum alkoxide may be diethylaluminum ethoxide.

The aluminoxane compound may be methylaluminoxane (MAO), ethylaluminoxane, modified methylaluminoxane (MMAO), n-propylaluminoxane, iso-propyl-aluminoxane, n-butylaluminoxane, sec-butylaluminoxane, iso-butylaluminoxane, t-butylaluminoxane, 1-pentyl-aluminoxane, 2-pentyl-aluminoxane, 3-pentyl-aluminoxane, iso-pentyl-aluminoxane, neopentylaluminoxane, or mixtures thereof.

The preferred co-catalyst is modified methylaluminoxane. The synthesis of modified methylaluminoxane may be carried out in the presence of other trialkylaluminum compounds in addition to trimethylaluminum. The products incorporate both methyl and alkyl groups from the added trialkylaluminum and are referred to as modified methyl aluminoxanes, MMAO. The MMAO may be more soluble in nonpolar reaction media, more stable to storage, have enhanced performance as a cocatalyst, or any combination of these. The performance of the resulting MMAO may be superior to either of the trialkylaluminum starting materials or to simple mixtures of the two starting materials. The added trialkylaluminum may be triethylaluminum, triisobutylaluminum or triisooctylaluminum. In one embodiment, the co-catalyst is MMAO, wherein preferably about 25% of the methyl groups are replaced with iso-butyl groups.

In one embodiment, the co-catalyst may be formed in situ in the reactor by providing the appropriate precursors into the reactor.

### Solvent

One or more solvents may be used in the reaction. The solvent(s) may be used to dissolve or suspend the catalyst or the co-catalyst and/or keep the ethylene dissolved. The solvent may be any solvent that can modify the solubility of any of these components or of reaction products. Suitable solvents include hydrocarbons, for example, alkanes, alkenes, cycloalkanes, and aromatics. Different solvents may be used in the process, for example, one solvent can be used for the catalyst and another for the co-catalyst. It is preferred for the solvent to have a boiling point that is not substantially similar to the boiling point of any of the alpha olefin products as this will make the product separation step more difficult.

### Aromatics

Aromatic solvents can be any solvent that contains an aromatic hydrocarbon, preferably having a carbon number of 6 to 20. These solvents may include pure aromatics, or mixtures of pure aromatics, isomers as well as heavier solvents, for example C₉ and C₁₀ solvents. Suitable aromatic solvents include benzene, toluene, xylene (including ortho-xylene, meta-xylene, para-xylene and mixtures thereof) and ethylbenzene.

### Alkanes

Alkane solvents may be any solvent that contains an alkyl hydrocarbon. These solvents may include straight chain alkanes and branched or iso-alkanes having from 3 to 20 carbon atoms and mixtures of these alkanes. The alkanes may be cycloalkanes. Suitable solvents include propane, isobutane, n-butane, butane (n-butane or a mixture of linear and branched C₄ acyclic alkanes), pentane (n-pentane or a mixture of linear and branched acyclic alkanes), hexane (n-hexane or a mixture of linear and branched C₆ acyclic alkanes), heptane (n-heptane or a mixture of linear and branched C₇ acyclic alkanes), octane (n-octane or a mixture of linear and branched C₈ acyclic alkanes) and isooctane. Suitable solvents also include cyclohexane and methylcyclohexane. In one embodiment, the solvent comprises C₆, C₇ and C₈ alkanes, that may include linear, branched and iso-alkanes.

### Catalyst System

The catalyst system may be formed by mixing together the ligand, the metal, the co-catalyst and optional additional compounds in a solvent. The feed may be present in this step.

In one embodiment, the catalyst system may be prepared by contacting the metal or metal compound with the ligand to form a catalyst precursor mixture and then contacting the catalyst precursor mixture with the co-catalyst in the reactor to form the catalyst system.

In some embodiments, the catalyst system may be prepared outside of the reactor vessel and fed into the reactor vessel. In other embodiments, the catalyst system may be formed in the reactor vessel by passing each of the components of the catalyst system separately into the reactor. In other embodiments, one or more catalyst precursors may be formed by combining at least two components outside of the reactor and then passing the one or more catalyst precursors into the reactor to form the catalyst system.

### Reaction Conditions

The oligomerization reaction is a reaction that converts the olefin feed in the presence of an oligomerization catalyst and a co-catalyst into a higher oligomer product stream.

### Temperature

The oligomerization reaction may be conducted over a range of temperatures of from -100 °C to 300 °C, preferably in the range of from 0 °C to 200 °C, more preferably in the range of from 50 °C to 150 °C and most preferably in the range of from 70 °C to 130 °C.

### Pressure

The oligomerization reaction may be conducted at a pressure of from 0.01 to 15 MPa and more preferably from 1 to 10 MPa.

The optimum conditions of temperature and pressure used for a specific catalyst system, to maximize the yield of oligomer, and to minimize the impact of competing reactions, for example dimerization and polymerization can be determined by one of ordinary skill in the art. The temperature and pressure are selected to yield a product slate with a K-factor in the range of from 0.40 to 0.90, preferably in the range of from 0.45 to 0.80, more preferably in the range of from 0.5 to 0.7.

### Residence Time

Residence times in the reactor of from 3 to 60 min have been found to be suitable, depending on the activity of the catalyst. In one embodiment, the reaction is carried out in the absence of air and moisture.

### Gas Phase, Liquid Phase or Mixed Gas-Liquid Phase

The oligomerization reaction can be carried out in the liquid phase or mixed gas-liquid phase, depending on the volatility of the feed and product olefins at the reaction conditions.

### Reactor type

The oligomerization reaction may be carried out in a conventional fashion. It may be carried out in a stirred tank reactor, wherein solvent, olefin and catalyst or catalyst precursors are added continuously to a stirred tank and solvent, product, catalyst, and unused reactant are removed from the stirred tank with the product separated and the unused reactant recycled back to the stirred tank.

In another embodiment, the oligomerization reaction may be carried out in a batch reactor, wherein the catalyst precursors and reactant olefin are charged to an autoclave or other vessel and after being reacted for an appropriate time, product is separated from the reaction mixture by conventional means, for example, distillation.

In another embodiment, the oligomerization reaction may be carried out in a gas lift reactor. This type of reactor has two vertical sections (a riser section and a downcomer section) and a gas separator at the top. The gas feed (ethylene) is injected at the bottom of the riser section to drive circulation around the loop (up the riser section and down the downcomer section).

In another embodiment, the oligomerization reaction may be carried out in a pump loop reactor. This type of reactor has two vertical sections, and it uses a pump to drive circulation around the loop. A pump loop reactor can be operated at a higher circulation rate than a gas lift reactor.

In another embodiment, the oligomerization reaction may be carried out in a once-through reactor. This type of reactor feeds the catalyst, co-catalyst, solvent and ethylene to the inlet of the reactor and/or along the reactor length and the product is collected at the reactor outlet. One example of this type of reactor is a plug flow reactor.

### Catalyst Deactivation

The higher oligomers produced in the oligomerization reaction contains catalyst from the reaction step. To stop further reactions that can produce byproducts and other undesired components, it is important to deactivate the catalyst downstream from the reactor.

In one embodiment, the catalyst is deactivated by addition of an acidic species having a pKₐ(aq) of less than 25. The deactivated catalyst can then be removed by water washing in a liquid/liquid extractor.

### Product Separation

The resulting alpha-olefins have a chain length of from 4 to 100 carbon atoms, preferably 4 to 30 carbon atoms and most preferably 4 to 20 carbon atoms. The alpha-olefins are even-numbered alpha-olefins.

The product olefins can be recovered by distillation or other separation techniques depending on the intended use of the products. The solvent(s) used in the reaction preferably have a boiling point that is different from the boiling point of any of the alpha-olefin products to make the separation easier.

In one embodiment, the distillation steps comprise columns for separating ethylene and the main linear alpha olefin products, for example, butene, hexene, and octene.

### Product qualities and characteristics

The products produced by the process may be used in a number of applications. The olefins produced by this process may have improved qualities as compared to olefins produced by other processes. In one embodiment, the butene, hexene and/or octene produced may be used as a comonomer in making polyethylene. In one embodiment, the octene produced may be used to produce plasticizer alcohols. In one embodiment, the decene produced may be used to produce polyalphaolefins. In one embodiment, the dodecene and/or tetradecene produced may be used to produce alkylbenzene and/or detergent alcohols. In one embodiment, the hexadecene and/or octadecene produced may be used to produce alkenyl succinates and/or oilfield chemicals. In one embodiment, the C20+ products may be used to produce lubricant additives and/or waxes.

### Recycle

A portion of any unreacted ethylene that is removed from the reactor with the products may be recycled to the reactor. This ethylene may be recovered in the distillation steps used to separate the products. The ethylene may be combined with the fresh ethylene feed or it may be fed separately to the reactor.

A portion of any solvent used in the reaction may be recycled to the reactor. The solvent may be recovered in the distillation steps used to separate the products.

### Oxygen

Contrary to the teachings of the prior art, the addition of oxygen to the oligomerization process has resulted in a significant increase in catalyst activity. Further, the addition of oxygen inhibits the self-limiting behavior of the catalyst when operating at temperatures above 110 °C. At these temperatures and without oxygen, the catalyst has high production for an initial time period, but then over time becomes self-limiting such that additional iron/ligand and MMAO does not result in an increase in activity. The examples provided below demonstrate these and other benefits associated with the addition of oxygen to the oligomerization reaction zone.

The oxygen may be added to the ethylene feed to the reaction zone or it may be added to the reaction zone separately from the ethylene feed. In one embodiment, the ethylene comprises oxygen. In another embodiment, the ethylene is treated to remove oxygen after which a specific concentration of oxygen is added to the ethylene before feeding the ethylene into the reaction zone.

The oxygen is added in a concentration to provide sufficient oxygen to the oligomerization reaction zone such that the production of alpha-olefins is at least 1.1 times the production of alpha-olefins under the same conditions but without oxygen, preferably at least 1.2 times the production, more preferably at least 1.3 times the production and most preferably at least 1.5 times the production.

The amount of oxygen fed to the oligomerization reaction zone may be determined in a number of different ways depending on the operation of the oligomerization reaction zone. In one embodiment, oxygen is fed to the oligomerization reaction zone at a molar ratio of oxygen to iron being fed to the oligomerization reaction zone of from 1:1 to 200:1. In other embodiments, the feeds to the oligomerization reaction zone of oxygen and iron are at a molar ratio of oxygen to iron of at least 1:1, at least 1.5:1, at least 2:1 or at least 3:1. In other embodiments, the feeds to the oligomerization reaction zone of oxygen and iron are at a molar ratio of oxygen to iron of at most 200:1, at most 100:1, at most 50:1, at most 20:1 or at most 6:1.

The oxygen in the feed to the oligomerization reaction zone may be within any range specified by one of the above lower limits and one of the above upper limits. For example, the feed to the oligomerization reaction zone of oxygen may be at a molar ratio of oxygen to iron of from 1:1 to 200:1, preferably of from 1.5:1 to 100:1, more preferably of from 2:1 to 50:1, most preferably of from 2:1 to 20:1. In other embodiments, the feed to the oligomerization reaction zone of oxygen may be at a molar ratio of oxygen to iron of from 2:1 to 6:1, preferably of from 3:1 to 6:1.

In another embodiment, the co-catalyst comprises MMAO and the oxygen feed supplied is measured with regards to the molar ratio of oxygen to aluminum in the MMAO. The aluminum in MMAO is reported on the vendor's certificate of analysis, and active aluminum is defined as the amount of aluminum in the co-catalyst that is active as AIR3. The active aluminum in the examples below is 39% of the total aluminum in MMAO.

In one embodiment, the feed to the oligomerization reaction zone of oxygen is at a molar ratio of oxygen to aluminum in the MMAO feed of less than 1:5. In another embodiment, the feed to the oligomerization reaction zone of oxygen is at a molar ratio of oxygen to aluminum in the MMAO feed of from 1:5 to 1:20.

In another embodiment, the oxygen feed to the oligomerization reaction zone is calculated on the basis of the contents of the reaction zone. In one embodiment, the feed to the oligomerization reaction zone of oxygen is at a concentration of from 0.2 to 200 ppmw, calculated based on the contents of the oligomerization reaction zone. In another embodiment, the feed to the oligomerization reaction zone of oxygen is at a concentration of from 0.5 to 100 ppmw. In a further embodiment, the feed to the oligomerization reaction zone of oxygen is at a concentration of from 1 to 60 ppmw. All of these are calculated based on the contents of the oligomerization reaction zone.

### Examples

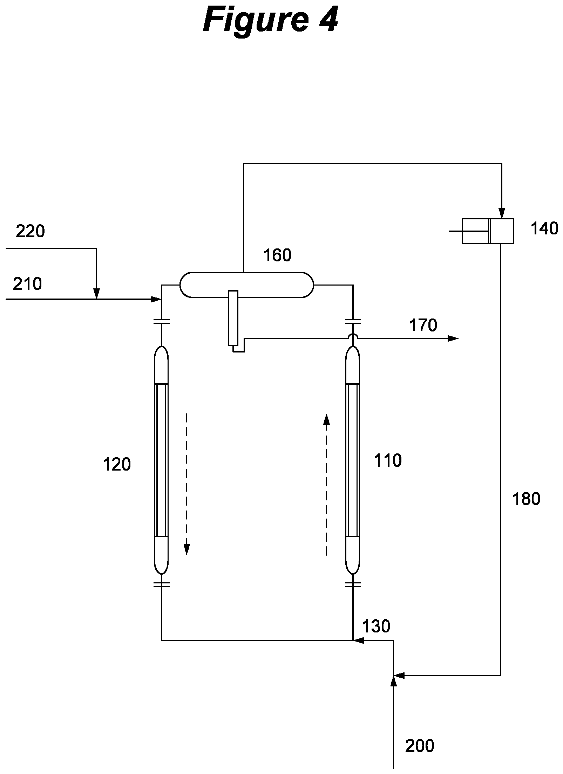

These examples were carried out in a gas-lift reactor depicted in Figure 4 and further described herein. The examples were all conducted as part of the same operational run of the gas-lift reactor, but the feed of the catalyst-ligand stream and the co-catalyst stream into the reaction zone were varied for each example.

Figure 4 depicts the ethylene oligomerization reactor that was operated with continuous feed as a gas-lift loop reactor to produce alpha olefins (AO). The reactor volume was 9.5 L and the typical circulation velocity is from 0.6 to 1.1 m/sec. Circulation for the gas lift reactor is provided by injecting ethylene at the bottom of the riser 110. The gas holdup in the riser creates a differential head pressure between the riser 110 and the downcomer 120 that drives liquid circulation down the downcomer and up the riser.

The riser and downcomer each are coaxial pipes with an outer heat exchanger shell for heat removal from the exothermic oligomerization reaction. The heat transfer fluid in the exchangers is water and each exchanger has an internal temperature indicator probe at the inlet and outlet as well as a mass flow controller to quantify the heat of reaction. Reactor temperature is controlled by a jacketed water heating system to preheat the reactor for startup or remove heat of reaction from the oligomerization reaction. The temperature of the gas lift reactor can be controlled from 60 to 99° C. The heating system is also able to operate in a melt out mode at a temperature of 121 to 154° C.

Ethylene feed is pretreated in a carbon bed, a molecular sieve bed, and then an oxygen removal bed (not shown) and then compressed to about 345 kPa above the reactor operating pressure and fed to the reactor through a control valve. The ethylene is supplied on pressure demand to maintain the reactor operating pressure from 2.8 MPa to 6.2 MPa. A regulated 0-18 kg/hr fresh ethylene feed 200 provides ethylene to the reaction zone by feeding at the reactor bottom through an injection nozzle 130. The ethylene recycle compressor 140 circulates ethylene for the gas lift and operates between 0.45 and 18 kg/hr.

Solvent feed is provided at a flow rate of 4.5 to 11.3 kg/hr. Solvent is fed through a diaphragm pump and then through two control valves before mixing with the catalyst feed solutions and entering the reactor. The solvent flow is divided between the two catalyst feed streams using the control valves.

The reactor can use separate feed lines for ligand, iron, and MMAO catalyst solutions fed to the reactor zone. In Figure 4, the ligand and iron are precomplexed and added as a single feed stream 210. The MMAO is added through line 220. Each catalyst stream is fed through an ISCO pump that is supplied by a catalyst supply feed vessel. The ISCO pump outlet operates at reactor pressure and the feed rate range for the pump is from 0.001 to 100 ml/min. MMAO and ligand/iron catalyst feeds are each blended with part of the total solvent recycle feed before entering the reactor.

The reactor top has an overhead separator 160 that allows for liquid to overflow into a heat traced pipe to control level. A downstream valve controls the level in the overflow pipe and this downstream product flow 170 is distilled to separate AO products from the solvent which is recycled back to the reactor. The liquid reactor outlet and downstream lines are heat traced with steam to maintain a temperature of 127° C to 160° C.

The gas phase that exits the top of the overhead separator goes through a cooler and then a gas/liquid separator to remove liquid upstream of the recycle compressor 140. This gas phase is recycled back to the reactor bottom via recycle line 180 and the liquid feeds forward to distillation.

SLPM : Standard liter per minute.For conversion in the SI units , the units indicated in 1bs correspond to :
6.5 lbs to 2.94835 kg, 7 lbs to 3.17515 kg, 11 lbs to 4.98952 kg, 14 lbs to 6.35029 kg, 15 lbs to 6.804 kg, 20 lbs to 9.0718 kg.

### Example 1

This example was conducted during a single run of the pilot plant. Examples 1a and 1b demonstrate the impact of oxygen addition in different ways that are more fully described for each example. The pilot plant was operated at 121 °C and 3.8 MPa. The catalyst concentration in 20 lbs/hr of solvent feed was 0.6 ppmw Fe, 58 ppmw Al from MMAO with a 200 Al/Fe mol/mol ratio.

### Example 1a

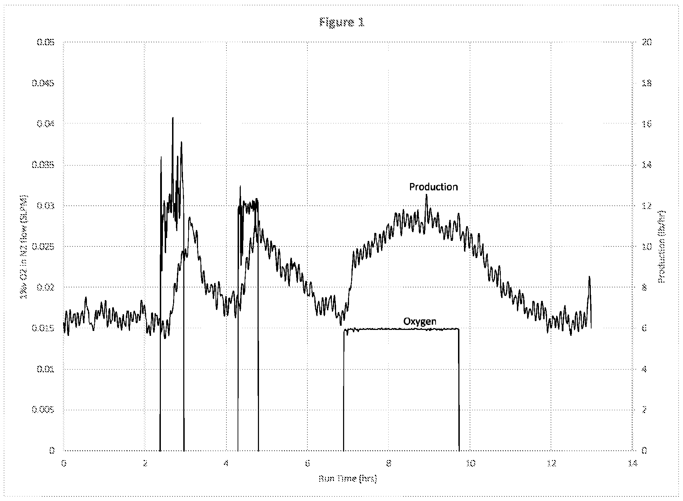

In this part of the run, a small flow of 1.0 vol. % oxygen in nitrogen into the pilot plant reactor was started and stopped a number of times to determine the effect on the production of alpha olefins. The amount of oxygen and the production of alpha olefins during the pilot plant test are shown in Figure 1. The pilot plant was started up without oxygen and the system was allowed to come to steady state. Oxygen was added and the production increased from about 6.5 lbs/hr to more than 11 lbs/hr. The oxygen was stopped, and production decreased to about 7 lbs/hr before the oxygen was restarted and production again increased to about 11 lbs/hr. The oxygen was stopped again, and production decreased to about 7 lbs/hr. The oxygen was restarted, and the production again increased to about 11 lbs/hr while oxygen was being added.

### Example 1b

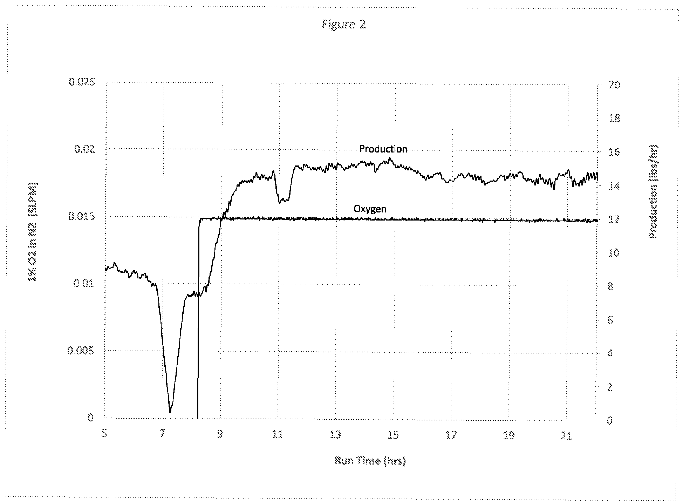

In this part of the run, a steady flow of oxygen (1.0 vol% oxygen in nitrogen) was added to the ethylene feed in the pilot plant reactor to further test the impact of adding oxygen to the reactor. The amount of oxygen and the production of alpha olefins during the pilot plant test are shown in Figure 2. This part of the run started without oxygen, but then 1.0 vol% oxygen in nitrogen was added at a rate of 0.015 SLPM and this flow was continued for over 10 hours. The molar concentration of iron feed was 0.1 mmol/hr compared to 0.4 mmol/hr of O₂ and 20 mmol/hr of Al from MMAO. The production increase from about 7 lbs/hr before the oxygen flow was started to about 15 lbs/hr after the oxygen was introduced and maintained that level of production for over 10 hours.

### Example 3

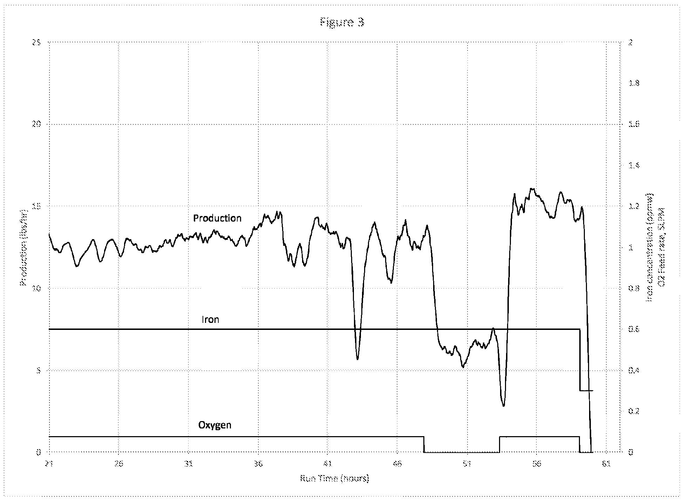

In this example, a further test was conducted to test the effect of oxygen. A steady flow of oxygen (0.1 vol% oxygen in nitrogen) was added to the pilot plant reactor to further test the impact of adding oxygen to the reactor. The pilot plant was operated at 121 °C and 5.2 MPa. The catalyst concentration in 20 lbs/hr solvent feed was 0.6 ppmw Fe, 29 ppmw Al from MMAO with a 100 Al/Fe mol/mol ratio. Oxygen was added at a rate of 0.075 SLPM of the 0.1 vol% oxygen in nitrogen. The molar concentration of iron feed was 0.1 mmol/hr compared to 0.2 mmol/hr of O₂ and 10 mmol/hr of Al from MMAO. The amount of oxygen and the production of alpha olefins during the pilot plant test are shown in Figure 3. While oxygen was added to the ethylene feed of the reactor, the production rate was about 14 lbs/hr. The oxygen was stopped, and the production decreased to about 7 lbs/hr. When oxygen was introduced again, the production increased to about 15 lbs/hr.

These examples demonstrate the effect that oxygen has on this reactor system, and that the presence of oxygen in the reactor system increases the production of valuable alpha olefins.

## Claims

1. A process for producing alpha-olefins comprising contacting an ethylene feed with an oligomerization catalyst system in an oligomerization reaction zone under oligomerization reaction conditions to produce a product stream comprising alpha-olefins wherein the catalyst system comprises an iron-ligand complex and a co-catalyst and the molar ratio of oxygen to iron being fed to the oligomerization reaction zone is of from 1:1 to 200:1.

2. The process of claim 1 wherein the co-catalyst comprises modified methyl aluminoxane (MMAO).

3. The process of any of claims 1-2 wherein the molar ratio of oxygen to iron being fed to the reaction zone is of from 1.5:1 to 100:1.

4. The process of any of claims 1-2 wherein the molar ratio of oxygen to iron being fed to the reaction zone is of from 2:1 to 50:1.

5. The process of any of claims 1-2 wherein the molar ratio of oxygen to iron being fed to the reaction zone is of from 2:1 to 20:1.

6. The process of any of claims 1-2 wherein the molar ratio of oxygen to iron being fed to the reaction zone is of from 2:1 to 6:1.

7. The process of any of claims 1-2 wherein the molar ratio of oxygen to iron being fed to the reaction zone is of from 3:1 to 6:1.

8. The process of any of claims 1-7 wherein the oligomerization reaction conditions comprises a temperature of from 70 to 130 °C.

9. The process of any of claims 1-8 wherein the ethylene feed comprises an amount of oxygen sufficient to provide the desired feed of oxygen to the reaction zone.

10. The process of any of claims 1-8 wherein the oxygen is added to the ethylene feed to provide oxygen to the reaction zone.

11. The process of any of claims 1-5 wherein oxygen is fed separately to the reaction zone.

12. A process for producing alpha-olefins comprising contacting an ethylene feed with an oligomerization catalyst system in an oligomerization reaction zone under oligomerization reaction conditions to produce a product stream comprising alpha-olefins wherein the catalyst system comprises an iron-ligand complex and a co-catalyst comprising aluminum and molar ratio of oxygen to aluminum in MMAO being fed to the oligomerization reaction zone is less than 1:5.

13. The process of claim 12 wherein the molar ratio of oxygen to aluminum in MMAO being fed to the oligomerization reaction zone is of from 1:5 to 1:20.

14. A process for producing alpha-olefins comprising contacting an ethylene feed with an oligomerization catalyst system in an oligomerization reaction zone under oligomerization reaction conditions to produce a product stream comprising alpha-olefins wherein the catalyst system comprises an iron-ligand complex and a co-catalyst and oxygen fed to the oligomerization reaction zone is at a concentration of from 0.2 to 200 ppmw, calculated based on the contents of the oligomerization reaction zone.

15. The process of claim 14 wherein the oxygen fed to the oligomerization reaction zone is at a concentration of from 0.5 to 100 ppmw, calculated based on the contents of the oligomerization reaction zone.

16. The process of claim 14 wherein the oxygen fed to the oligomerization reaction zone is at a concentration of from 1 to 60 ppmw, calculated based on the contents of the oligomerization reaction zone.

## Patentansprüche

1. Verfahren zum Herstellen von Alpha-Olefinen, umfassend ein Inkontaktbringen einer Ethylenzufuhr mit einem Oligomerisierungskatalysatorsystem in einer Oligomerisierungsreaktionszone unter Oligomerisierungsreaktionsbedingungen, um einen Produktstrom zu erzeugen, umfassend Alpha-Olefine, wobei das Katalysatorsystem einen Eisen-Ligand-Komplex und einen Co-Katalysator umfasst und das Molverhältnis von Sauerstoff zu Eisen, das der Oligomerisierungsreaktionszone zugeführt wird, von 1 : 1 bis 200 : 1 beträgt.

2. Verfahren nach Anspruch 1, wobei der Co-Katalysator modifiziertes Methylaluminoxan (MMAO) umfasst.

3. Verfahren nach einem der Ansprüche 1 bis 2, wobei das Molverhältnis von Sauerstoff zu Eisen, das der Reaktionszone zugeführt wird, von 1,5 : 1 bis 100 : 1 beträgt.

4. Verfahren nach einem der Ansprüche 1 bis 2, wobei das Molverhältnis von Sauerstoff zu Eisen, das der Reaktionszone zugeführt wird, von 2 : 1 bis 50 : 1 beträgt.

5. Verfahren nach einem der Ansprüche 1 bis 2, wobei das Molverhältnis von Sauerstoff zu Eisen, das der Reaktionszone zugeführt wird, von 2 : 1 bis 20 : 1 beträgt.

6. Verfahren nach einem der Ansprüche 1 bis 2, wobei das Molverhältnis von Sauerstoff zu Eisen, das der Reaktionszone zugeführt wird, von 2 : 1 bis 6 : 1 beträgt.

7. Verfahren nach einem der Ansprüche 1 bis 2, wobei das Molverhältnis von Sauerstoff zu Eisen, das der Reaktionszone zugeführt wird, von 3 : 1 bis 6 : 1 beträgt.

8. Verfahren nach einem der Ansprüche 1 bis 7, wobei die Oligomerisierungsreaktionsbedingungen eine Temperatur von 70 bis 130 °C umfassen.

9. Verfahren nach einem der Ansprüche 1 bis 8, wobei die Ethylenzufuhr eine Sauerstoffmenge umfasst, die ausreicht, um die gewünschte Sauerstoffzufuhr der Reaktionszone bereitzustellen.

10. Verfahren nach einem der Ansprüche 1 bis 8, wobei der Sauerstoff der Ethylenzufuhr zugesetzt wird, um der Reaktionszone Sauerstoff bereitzustellen.

11. Verfahren nach einem der Ansprüche 1 bis 5, wobei Sauerstoff der Reaktionszone separat zugeführt wird.

12. Verfahren zum Herstellen von Alpha-Olefinen, umfassend das Inkontaktbringen einer Ethylenzufuhr mit einem Oligomerisierungskatalysatorsystem in einer Oligomerisierungsreaktionszone unter Oligomerisierungsreaktionsbedingungen, um einen Produktstrom zu erzeugen, umfassend Alpha-Olefine, wobei das Katalysatorsystem einen Eisen-Ligand-Komplex und einen Co-Katalysator umfasst, umfassend Aluminium, und das Molverhältnis von Sauerstoff zu Aluminium in dem MMAO, das der Oligomerisierungsreaktionszone zugeführt wird, weniger als 1 : 5 beträgt.

13. Verfahren nach Anspruch 12, wobei das Molverhältnis von Sauerstoff zu Aluminium in dem MMAO, das der Oligomerisierungsreaktionszone zugeführt wird, von 1 : 5 bis 1 : 20 beträgt.

14. Verfahren zum Herstellen von Alpha-Olefinen, umfassend das Inkontaktbringen einer Ethylenzufuhr mit einem Oligomerisierungskatalysatorsystem in einer Oligomerisierungsreaktionszone unter Oligomerisierungsreaktionsbedingungen, um einen Produktstrom zu erzeugen, umfassend Alpha-Olefine, wobei das Katalysatorsystem einen Eisen-Ligand-Komplex und einen Co-Katalysator umfasst, und Sauerstoff, der der Oligomerisierungsreaktionszone zugeführt wird, in einer Konzentration von 0,2 bis 200 ppmw vorliegt, berechnet basierend auf den Inhalten der Oligomerisierungsreaktionszone.

15. Verfahren nach Anspruch 14, wobei der Sauerstoff, der der Oligomerisierungsreaktionszone zugeführt wird, in einer Konzentration von 0,5 bis 100 ppmw vorliegt, berechnet basierend auf den Inhalten der Oligomerisierungsreaktionszone.

16. Verfahren nach Anspruch 14, wobei der Sauerstoff, der der Oligomerisierungsreaktionszone zugeführt wird, in einer Konzentration von 1 bis 60 ppmw vorliegt, berechnet basierend auf den Inhalten der Oligomerisierungsreaktionszone.

## Revendications

1. Procédé permettant de produire des alpha-oléfines comprenant la mise en contact d'une alimentation d'éthylène avec un système de catalyseur d'oligomérisation dans une zone de réaction d'oligomérisation dans des conditions de réaction d'oligomérisation pour produire un courant de produit comprenant des alpha-oléfines dans lequel le système de catalyseur comprend un complexe fer-ligand et un co-catalyseur et le rapport molaire de l'oxygène au fer étant alimenté à la zone de réaction d'oligomérisation va de 1:1 à 200:1.

2. Procédé selon la revendication 1 dans lequel le co-catalyseur comprend du méthyl-aluminoxane modifié (MMAO).

3. Procédé selon l'une quelconque des revendications 1 à 2 dans lequel le rapport molaire de l'oxygène au fer étant alimenté à la zone de réaction va de 1,5:1 à 100:1.

4. Procédé selon l'une quelconque des revendications 1 à 2 dans lequel le rapport molaire de l'oxygène au fer étant alimenté à la zone de réaction va de 2:1 à 50:1.

5. Procédé selon l'une quelconque des revendications 1 à 2 dans lequel le rapport molaire de l'oxygène au fer étant alimenté à la zone de réaction va de 2:1 à 20:1.

6. Procédé selon l'une quelconque des revendications 1 à 2 dans lequel le rapport molaire de l'oxygène au fer étant alimenté à la zone de réaction va de 2:1 à 6:1.

7. Procédé selon l'une quelconque des revendications 1 à 2 dans lequel le rapport molaire de l'oxygène au fer étant alimenté à la zone de réaction va de 3:1 à 6:1.

8. Procédé selon l'une quelconque des revendications 1 à 7 dans lequel les conditions de réaction d'oligomérisation comprennent une température allant de 70 à 130 °C.

9. Procédé selon l'une quelconque des revendications 1 à 8 dans lequel l'alimentation d'éthylène comprend une quantité d'oxygène suffisante pour fournir l'alimentation souhaitée d'oxygène à la zone de réaction.

10. Procédé selon l'une quelconque des revendications 1 à 8 dans lequel l'oxygène est ajouté à l'alimentation d'éthylène pour fournir de l'oxygène à la zone de réaction.

11. Procédé selon l'une quelconque des revendications 1 à 5 dans lequel de l'oxygène est alimenté séparément à la zone de réaction.

12. Procédé permettant de produire des alpha-oléfines comprenant la mise en contact d'une alimentation d'éthylène avec un système de catalyseur d'oligomérisation dans une zone de réaction d'oligomérisation dans des conditions de réaction d'oligomérisation pour produire un courant de produit comprenant des alpha-oléfines dans lequel le système de catalyseur comprend un complexe fer-ligand et un co-catalyseur comprenant de l'aluminium et un rapport molaire de l'oxygène à l'aluminium dans le MMAO étant alimenté à la zone de réaction d'oligomérisation est inférieur à 1:5.

13. Procédé selon la revendication 12 dans lequel le rapport molaire de l'oxygène à l'aluminium dans le MMAO étant alimenté à la zone de réaction d'oligomérisation va de 1:5 à 1:20.

14. Procédé permettant de produire des alpha-oléfines comprenant la mise en contact d'une alimentation d'éthylène avec un système de catalyseur d'oligomérisation dans une zone de réaction d'oligomérisation dans des conditions de réaction d'oligomérisation pour produire un courant de produit comprenant des alpha-oléfines dans lequel le système de catalyseur comprend un complexe fer-ligand et un co-catalyseur et l'oxygène alimenté à la zone de réaction d'oligomérisation est à une concentration allant de 0,2 à 200 ppm en poids, calculée en fonction du contenu de la zone de réaction d'oligomérisation.

15. Procédé selon la revendication 14 dans lequel l'oxygène alimenté à la zone de réaction d'oligomérisation est à une concentration allant de 0,5 à 100 ppm en poids, calculée en fonction du contenu de la zone de réaction d'oligomérisation.

16. Procédé selon la revendication 14 dans lequel l'oxygène alimenté à la zone de réaction d'oligomérisation est à une concentration allant de 1 à 60 ppm en poids, calculée en fonction du contenu de la zone de réaction d'oligomérisation.
